# EUROPEAN PATENT SPECIFICATION

(11) **EP 1 427 403 B1**
(45) Date of publication and mention of the grant of the patent: **28.12.2005**
(21) Application number: 02777129.4
(22) Date of filing: 17.09.2002
(51) Int. Cl.: A61K 31/19, A61P 35/00

(54) **Valproic acid for the treatment of breast cancer, colon cancer, head and neck cancer, small cell lung carcinoma and cancer of the blood cells in combination with irradiation**
Valproinsäure zur Behandlung von Brustkrebs, Dickdarmkrebs, Kopf-Hals-Krebs, kleinzelligem Lungenkarzinom und Blutzellenkrebs in Kombination mit Bestrahlung
Acide valproique pour le traitement des cancers du sein, du colon, de la tête et du cou, du carcinome du poumon à petites cellules et du cancer de cellules sanguines en combinaison avec l'irradiation

(30) Priority: 18.09.2001 EP 01121722
(43) Date of publication of application: 16.06.2004
(60) Divisional application: 05019659.1
(73) Proprietor: G2M Cancer Drugs AG, 60596 Frankfurt am Main (DE)
(72) Inventor: GRONER, Bernd, 60322 Frankfurt am Main (DE); HEINZEL, Thorsten, 60529 Frankfurt am Main (DE); HENTSCH, Bernd, 47239 Duisburg (DE); WELS, Winfried, Stephan, 60599 Frankfurt (DE); HERRLICH, Peter, A., 76229 Karlsruhe (DE); MINUCCI, Saverio, I-20090 Opera (IT); PELICCI, Pier, Giuseppe, I-20090 Opera (IT); GÖTTLICHER, Martin, 76297 Stutensee (DE)
(74) Representative: Keller, Günter
(86) International application number: PCT/EP2002/010419
(87) International publication number: WO 2003/024442

(56) References cited:
- EP-A- 0 360 205
- EP-A- 0 412 211
- EP-A- 0 632 008
- EP-A- 1 170 008
- WO-A-01/47533
- US-A- 5 672 746
- US-A- 5 939 455
- US-A- 6 028 102
- US-A- 6 110 955
- US-B1- 6 207 147
- AIKEN TC, COLLIN RC: "A possible anti-emetic role for sodium valproate in cytotoxic chemotherapy" BRITISH JOURNAL OF HAEMATOLOGY, vol. 89, no. 4, 4 April 1995 (1995-04-04), pages 903-904, XP008004839
- DRIEVER PH, KNÜPFER MM, CINATL J, WOLFF JEA: "Valproic acid for the treatment of pediatric malignant glioma" KLINISCHE PAEDIATRIE, vol. 211, July 1999 (1999-07), pages 323-328, XP000997401
- KNÜPFER MM, HERN IZ-DRIEVER P, POPPENBORG H, WOLFF JEA, CINATL J: "Valproic Acid Inhibits Proliferation and Changes Expression of CD44 and CD56 of Malignant Glioma Cells in Vitro" ANTICANCER RESEARCH, vol. 18, no. 5A, 1998, pages 3585-3589, XP008004824
- YAO C-P, MATHER GG, STEPHENS JR, LEVY RH: "Cytotoxicity Induced by the Combination of Valproic Acid and Tumor Necrosis Factor-alpha" BIOCHEMICAL PHARMACOLOGY, vol. 58, 1999, pages 455-459, XP002203825
- VAMECQ J ET AL: "PRELIMINARY STUDIES ABOUT NOVEL STRATEGIES TO REVERSE CHEMORESISTANCE TO ADRIAMYCIN REGARDING GLUTATHIONE METABOLISM, PEROXISOMAL AND EXTRAPEROXISOMAL HYDROPEROXIDE AND VALPROIC ACID METABOLIC PATHWAYS" BIOLOGY OF THE CELL, ELSEVIER, PARIS, FR, vol. 77, 1993, pages 17-26, XP008004943 ISSN: 0248-4900
- FISCHKOFF S A ET AL: "INDUCTION OF NEUTROPHILIC DIFFERENTIATION OF HUMAN PROMYELOCYTIC LEUKEMIC CELLS BY BRANCHED-CHAIN CARBOXYLIC ACID ANTICONVULSANT DRUGS" JOURNAL OF BIOLOGICAL RESPONSE MODIFIERS, RAVEN PRESS, NEW YORK, NY, US, vol. 3, no. 2, April 1984 (1984-04), pages 132-137, XP000997488 ISSN: 0732-6580
- COURAGE-MAGUIRE C, BACON CL, NAU H, REGAN CM: "Correlation of in vitro anti-proliferative potential with in vivo teratogenicity in a series of valproate analogues" INT J DEVEL NEUROSCIENCE, vol. 15, no. 1, 1997, pages 37-43, XP002224044
- NORDENBERG J ET AL: "EFFECTS OF PSYCHOTROPIC DRUGS ON CELL PROLIFERATION AND DIFFERENTIATION" BIOCHEMICAL PHARMACOLOGY, PERGAMON, OXFORD, GB, vol. 58, no. 8, 15 October 1999 (1999-10-15), pages 1229-1236, XP001027786 ISSN: 0006-2952
- CINATL J JR, CINATL J, SCHOLZ M, HERN IZ-DRIEVER P, HENRICH D, KABICKOVA H, VOGEL J-U, DOERR HW, KORNHUBER B: "Antitumor activity of sodium valproate in cultures of human neuroblastoma cells" ANTI-CANCER DRUGS, vol. 7, no. 7, July 1996 (1996-07), pages 766-773, XP008004842
- CINATL J ET AL: "SODIUM VALPROATE INHIBITS IN VIVO GROWTH OF HUMAN NEUROBLASTOMA CELLS" ANTI-CANCER DRUGS, OXFORD, GB, vol. 8, no. 10, November 1997 (1997-11), pages 958-963, XP000997406 ISSN: 0959-4973
- TITTLE T V ET AL: "EFFECT OF ANTIEPILEPTIC DRUGS ON GROWTH OF MURINE LYMPHOID TUMOR CELLS IN SINGLE-CELL CULTURE" EPILEPSIA, RAVEN PRESS LTD., NEW YORK, US, vol. 33, no. 4, July 1992 (1992-07), pages 729-735, XP000997403 ISSN: 0013-9580
- DATABASE MEDLINE [Online] November 1978 (1978-11) MORI H ET AL: "[Effect of alpha-mercaptopropionylglycine (alpha-MPG) and sodium dipropylacetate (DPA) on antibody formation (IV). Tumor immunity (author's transl)]" Database accession no. NLM374206 XP002166105 & NIPPON YAKURIGAKU ZASSHI. JAPANESE JOURNAL OF PHARMACOLOGY. JAPAN NOV 1978, vol. 74, no. 8, November 1978 (1978-11), pages 907-923, ISSN: 0015-5691
- LETIZIA C ET AL: "REDUCTION OF AN ACTH SECRETING ADENOMA IN A PATIENT WITH NELSON'S SYNDROME CHRONICALLY TREATED WITH SODIUM VALPROATE" EUROPEAN JOURNAL OF INTERNAL MEDICINE, ELSEVIER, AMSTERDAM,, NL, vol. 5, no. 4, October 1994 (1994-10), pages 325-328, XP000997468 ISSN: 0953-6205
- GROSSMAN SA, HOCHBERG F, FISHER J, CHEN TL, KIM L, GREGORY R, GROCHOW LB, PIANTADOSI S: "Increased 9-aminocamptothecin dose requirements in patients on anticonvulsants" CANCER CHEMOTHER. PHARMACOL., vol. 42, 1998, pages 118-126, XP002202988
- STÄNDER M, DICHGANS J, WELLER M: "Anticonvulsant drugs fail to modulate chemotherapy-induced cytotoxicity and growth inhibition of human malignant glioma cells" JOURNAL OF NEURO-ONCOLOGY, vol. 37, no. 3, May 1998 (1998-05), pages 191-198, XP008004836
- TITTLE TV, SCHAUMANN BA, RAINEY JE, TAYLOR K: "Segregation of the Growth Slowing Effects of Valproic Acid from Phenyltoin and Carbamazepine on Lymphoid Tumor Cells" LIFE SCIENCES, vol. 50, no. 14, 1992, pages PL79-PL83, XP008005297
- LAMPEN A ET AL: "NEW MOLECULAR BIOASSAYS FOR THE ESTIMATION OF THE TERATOGENIC POTENCY OF VALPROIC ACID DERIVATIVES IN VITRO: ACTIVATION OF THE PEROXISOMAL PROLIFERATOR-ACTIVATED RECEPTOR (PPARDELTA)" TOXICOLOGY AND APPLIED PHARMACOLOGY, ACADEMIC PRESS, SAN DIEGO, CA,, US, vol. 160, no. 3, 1 November 1999 (1999-11-01), pages 238-249, XP000987176 cited in the application
- VAMECQ J ET AL: "PEROXISOME PROLIFERATOR-ACTIVATED RECEPTORS (PPARS) AND THEIR IMPLICATIONS IN DISEASES" CURRENT OPINION IN ENDOCRINOLOGY AND DIABETES, PHILADELPHIA, PA, US, vol. 7, no. 1, 2000, pages 8-18, XP000909179 ISSN: 1068-3097
- VAMECQ J ET AL: "Medical significance of peroxisome proliferator-activated receptors" LANCET, vol. 354, no. 9173, 10 July 1999 (1999-07-10), pages 141-148, XP004266618 ISSN: 0140-6736
- RAYNER A A ET AL: "LYMPHOKINE-ACTIVATED KILLER (LAK) CELLS ANALYSIS OF FACTORS RELEVANT TO THE IMMUNOTHERAPY OF HUMAN CANCER" CANCER, AMERICAN CANCER SOCIETY, PHILADELPHIA, PA, US, vol. 55, no. 3, 15 March 1985 (1985-03-15), pages 1327-1333, XP000108155 ISSN: 0008-543X

## Description

The present invention relates to the use of the drug valproic acid and pharmaceutically acceptable salts thereof for a sensitizing treatment of human cancers in combination with established therapeutic principles. The invention also relates to the use of those compounds for the treatment of tumor metastases and minimal residual disease. The invention includes the manufacture of a clinically used medicament for the treatment of human cancers.

Local remodeling of chromatin and dynamic changes in nucleosomal packaging of DNA are key steps in the regulation of gene expression and consequently affect proper cell function, differentiation and proliferation. One of the most important mechanisms determining the activity of target genes is the posttranslational modification of the N-terminal tails of core histones by acetylation and subsequent changes in chromatin structure (Davie, 1998, Curr Opin Genet Dev 8, 173-8; Kouzarides, 1999, Curr Opin Genet Dev 9, 40-8; Strahl and Allis, 2000, Nature 403, 41-4). Acetylation of lysine residues, predominantly in histones H3 and H4, is mediated by enzymes with histone acetyltransferase (HAT) activity. Conversely, acetyl groups are removed from ε-N-acetyl-lysine by histone deacetylases (HDACs). Both, HAT and HDAC activities can be recruited to target genes in complexes with sequence specific transcription factors and their cofactors. Nuclear receptors of the steroid/retinoid receptor superfamily such as retinoic acid receptor or thyroid hormone receptor are prototypical examples of transcription factors recruiting HAT and HDAC-associated cofactors depending on their status of activation by an appropriate ligand. In the absence of ligand these nuclear receptors interact with corepressors, e.g. N-CoR and SMRT. The corepressors form large protein complexes containing histone deacetylases and thereby inhibit transcription (Pazin and Kadonaga, 1997; Cell 89, 325-8). Upon ligand binding the corepressor complex dissociates and is replaced by coactivator proteins, e.g. SRC-1 and CBP, which exist in multiprotein complexes harboring histone acetyltransferase activity. The ligand-induced switch of nuclear receptors from repression to activation thus reflects the exchange of corepressor and coactivator complexes with antagonistic enzymatic activities (Glass and Rosenfeld, 2000, Genes Dev 14, 121-41). Intriguingly, many other transcription factors such as Mad-1, BCL-6, and ETO 4 (Pazin and Kadonaga, 1997, Cell 89, 325-8; Huynh and Bardwell, 1998, Oncogene 17, 2473-84; Wang, J. et al., 1998, Proc Natl Acad Sci U S A 95, 10860-5) have also been shown to assemble HDAC-dependent transcriptional repressor complexes, indicating that this is a common mechanism of gene regulation.

Mammalian histone deacetylases can be divided into three subclasses (Cress and Seto, 2000, J Cell Physiol 184, 1-16; Gray and Ekstrom 2001, Exp Cell Res 262, 75-83). Class I enzymes are homologues of the yeast RPD3 protein and include the mammalian HDAC1, HDAC2, HDAC3 and HDAC8 enzymes with molecular masses ranging from 42 to 55 kDa. Class II histone deacetylases HDAC4, HDAC5, HDAC6 and HDAC7 are larger proteins (about 120 to 130 kDa) which are related to the yeast HDA1 protein. Recently, a third class of histone deacetylases with homology to the yeast SIR2 protein and several putative mammalian members has been identified (Imai et al., 2000, Nature 403, 795-800). Presently, it is still unclear to which extent these HDACs exert isoenzyme-specific or redundant functions. Further studies including gene deletion analysis are therefore required to elucidate the biological roles of each of these enzymes.

Histone deacetylases bind to many different proteins and usually exist in large complexes within the cell. Many of the associated proteins seem to be involved in either targeting HDACs to their substrates or to transcriptional repressors. For example, the Rb-associated proteins RbAP46 and RbAP48 are usually considered as integral parts of the HDAC enzyme complex responsible for the recognition of nucleosomal substrates (Taunton et al., 1996, Science 272, 408-11; Verreault et al., 1996, Cell 87, 95-104). The corepressors N-CoR, SMRT and Sin3 on the other hand are bridging factors required for the recruitment of HDACs to transcription factors (Pazin and Kadonaga, 1997, Cell 88, 737-40). Histone deacetylases are also components of the nucleosome remodeling and deacetylase (NuRD) complex which also contains RbAP46 and RbAP48, Mi-2 and MTA2 (Zhang, Y. et al., 1999, Genes Dev 13, 1924-35). Given the large number of HDAC isoenzymes and interacting proteins it is conceivable that complex composition could modulate substrate specificity and target HDACs even to non-histone proteins.

Inappropriate repression of genes required for cell differentiation has been linked to several forms of cancer and in particular to acute leukemia. In acute promyelocytic leukemia (APL) patients, RAR fusion proteins resulting from chromosomal translocations involve either the promyelocytic leukemia protein (PML) or the promyelocytic zinc finger protein (PLZF) (de The, 1996, Faseb J 10, 955-60). Both fusion proteins can interact with components of the corepressor complex. The addition of high doses of all-trans retinoic acid, however, dismisses the corepressor complex only from PML-RAR, but not from PLZF-RAR (Grignani et al., 1998, Nature 391, 815-8; Guidez et al., 1998, Blood 91, 2634-42; He et al., 1998, Nat Genet 18, 126-35; Lin et al., 1998, Nature 391, 811-4). These findings provide an explanation why PML-RAR APL patients usually achieve complete remission upon retinoic acid treatment whereas PLZF-RAR APL patients respond very poorly to this therapy. The hypothesis that corepressor-mediated aberrant repression may be causal for pathogenesis in APL is supported by the finding that inhibitors of corepressor-associated HDAC activity are capable of overcoming the differentiation block in cells containing the PLZF-RAR fusion protein.

In a frequent form of acute myeloid leukemia (AML), the translocation t(8;21) results in the AML1/ETO fusion protein, in which the transactivation domain of transcription factor AML1 is replaced by almost the entire ETO protein. The translocation partner ETO has been reported to interact with N-CoR, SMRT, mSin3 and HDACs (Lutterbach et al., 1998, Mol Cell Biol 18, 7176-84; Gelmetti et al., 1998, Mol Cell Biol 18, 7185-91; Wang et al., 1998, Proc Natl Acad Sci U S A 95, 10860-5; Hildebrand et al., 2001, J Biol Chem 276, 9889-95). Thus, the fusion protein recruits corepressor complexes containing HDAC activity instead of coactivators. Recent reports indicate that the oncogenic potential and transcriptional repressor activity of the translocation product AML1/ETO requires oligomerization (Minucci et al., 2000, Mol Cell 5, 811-20). In non-Hodgkin's lymphoma, translocations and point mutations frequently lead to overexpression of the BCL-6 oncogene product which has been implicated in the control of B-cell proliferation. Since BCL-6 is a transcription factor which has been shown to interact with the corepressors N-CoR and SMRT, aberrant repression as in acute leukemias could also be involved in the pathogenesis of non-Hodgkin's lymphoma (Huynh and Bardwell, 1998, Oncogene 17, 2473-84).

Mutations in a nuclear hormone receptor have also been implicated as causal agents in the syndrome of Resistance to Thyroid Hormone (RTH), an endocrine human genetic disease characterized by a disruption in both, negative-feedback regulation and positive regulation by T₃. Diverse dominant negative mutations in the thyroid hormone receptor beta (TRβ) gene causing constitutive binding of corepressors and associated HDACs are the molecular basis of RTH (Yoh et al., 1997, Mol Endocrinol 11, 470-80; Yoh and Privalsky 2000, Mol Cell Endocrinol 159, 109-24).

Since pathogenesis in acute leukemia and non-Hodgkin's lymphoma is associated with the aberrant repression of genes required for cell differentiation it is plausible that this mechanism could also be relevant for many additional types of cancer including solid tumors. Currently, the molecular basis of many neoplasias is still largely unexplored. Due to the link between transcriptional repression and the recruitment of histone deacetylases, inhibitors of this enzymatic activity can be expected to reverse repression and to induce reexpression of differentiation inducing genes. Therefore, HDAC inhibitors are potentially promising candidate drugs for differentiation therapy of cancer and the treatment of certain endocrine diseases.

The clinical benefits of HDAC inhibition and their implications for re-differentiation therapy are currently being investigated in several locations. A PML-RAR patient who had experienced multiple relapses after treatment with retinoic acid and chemotherapy has been treated with the HDAC inhibitor phenylbutyrate, resulting in complete remission of the leukemia (Warrell et al., 1998, J Natl Cancer Inst 90, 1621-4). The result of this initial study suggests that high doses of HDAC inhibitors need not to be permanently sustained in order to achieve a clinical response. Phase II studies in cancer patients will serve as proof of principle for the effectiveness of HDAC inhibitors in therapy.

Recently, it was discovered that the antiepileptic drug valproic acid (VPA, 2-propylpentanoic acid) acts as an inhibitor of histone deacetylases (PCT/EP01/07704; Phiel et al., 2001, J Biol Chem, in press). This activity can be separated by appropriate modifications of the VPA molecule from the hitherto therapeutically exploited antiepileptic activity (PCT/EP01/07704).

Valproic acid has multiple biological activities which depend on different molecular mechanisms of action:
- VPA is an antiepileptic drug.
- VPA is teratogenic. When used as an antiepileptic drug during pregnancy VPA can induce birth defects (neural tube closure defects and other malformations) in a few percent of born children. In mice, VPA is teratogenic in the majority of mouse embryos when properly dosed.
- VPA activates a nuclear hormone receptor (PPARδ). Several additional transcription factors are also derepressed but some factors are not significantly derepressed (glucocorticoid receptor, PPARα).
- VPA occasionally causes hepatotoxicity, which may depend on poorly metabolized esters with coenzyme A.

The use of VPA derivatives allowed to determine that the different activities are mediated by different molecular mechanisms of action. Teratogenicity and antiepileptic activity follow different modes of action because compounds could be isolated which are either preferentially teratogenic or preferentially antiepileptic (Nau et al., 1991, Pharmacol. Toxicol. 69, 310-321). Activation of PPARδ was found to be strictly correlated with teratogenicity (Lampen et al., 1999, Toxicol. Appl. Pharmacol. 160, 238-249) suggesting that, both, PPARδ activation and teratogenicity require the same molecular activity of VPA. Also, differentiation of F9 cells strictly correlated with PPARδ activation and teratogenicity as suggested by Lampen et al., 1999, and documented by the analysis of differentiation markers (Werling et al., 2001, Mol. Pharmacol. 59, 1269-1276). It was shown, that PPARδ activation is caused by the HDAC inhibitory activity of VPA and its derivatives (PCT/EP01/07704). Furthermore it was shown that the established HDAC inhibitor TSA activates PPARδ and induces the same type of F9 cell differentiation as VPA. From these results we conclude that not only activation of PPARδ but also induction of F9 cell differentiation and teratogenicity of VPA or VPA derivatives are most likely caused by HDAC inhibition.

Antiepileptic and sedating activities follow different structure activity relationships and thus obviously depend on a primary VPA activity distinct from HDAC inhibition. The mechanism of hepatotoxicity is poorly understood and it is unknown whether it is associated with formation of the VPA-CoA ester. HDAC inhibition, however, appears not to require CoA ester formation.

Today, tumor therapies are known which consist of the combinatorial treatment of patients with more than one anti-tumor therapeutic reagent. Examples are the combined use of irradiation treatment together with chemotherapeutic and/or cytotoxic reagents and more recently the combination of irradiation treatment with immunological therapies such as the use of tumor cell specific therapeutic antibodies. However, the possibility to combine individual treatments with each other in order to identify such combinations which are more effective than the individual approaches alone, requires extensive pre-clinical and clinical testing. It is not possible to predict which combinations show an additive or even synergistic effect. Besides the aim to increase the therapeutic efficacy, another purpose is the potential decrease of the doses of the individual components in the resulting combinations in order to decrease unwanted or harmful side effects caused by higher doses of the individual components.

The present invention aims at providing a medicament which is useful for the treatment of human cancer.
To this end it was now surprisingly found that VPA has unexpected beneficial effects when used for the treatment of potentially many different types of human cancer in combination with a whole variety of other anti-tumor therapies which are individually based on strikingly different modes of action. Thus, the potential therapeutic use of VPA as a component of many anti-tumor drug combinations may not be limited to combinations with drugs having particular molecular mechanisms. This in fact may render VPA a drug to be combined with the majority of existing anti-tumor approaches. Here, the precise mode of action which is employed by VPA is not fully understood, but its differentiation inducing potential may be the basis to sensitize tumor cells for the activity of such a wide range of anti-tumor drugs. This surprisingly broad potential of VPA is expected to be based on its activity as an inhibitor of specific sets of enzymes having HDAC activity.

The present invention relates to the use of VPA and pharmaceutically acceptable salts thereof for the manufacture of a medicament for the treatment of human cancer in combination with irradiation treatment wherein the human cancer is selected from the group consisting of breast cancer, colon cancer, head and neck cancer, small cell lung carcinoma and cancer of blood cells. The anti-tumoral activity of such combinatorial treatments compared to the use of each component alone can thus be increased and - if desired - the doses of the individual components of such combinatorial treatments may be lowered in order to decrease unwanted side effects related to individual drugs.

As used herein, the term "combinatorial treatment" refers to a treatment of an individual with at least two different therapeutic agents. According to the invention, the individual is treated with valproic acid or a pharmaceutically acceptable salt thereof which constitutes the first therapeutic agent. The second therapeutic agent is radiation therapy. A combinatorial treatment may include a third or even further therapeutic agent. In accordance with the invention valproic acid or the pharmaceutically acceptable salt thereof and the second and optionally further therapeutic agent can be administered simultaneously, or VPA or a pharmaceutically acceptable salt thereof can be administered prior to or after the second therapeutic agent. Administration of VPA or a pharmaceutically acceptable salt thereof prior to the second therapeutic agent or simultaneous administration is preferred. Administration (simultaneously or at a different time) can be done systematically or topically as determined by the indication. VPA or a pharmaceutically acceptable salt thereof can be administered to a cancer patient pre- or post-radiation therapy to treat or ameliorate the effects of cancer. When the first and second therapeutic agent are applied at a different time, the time between the two treatments is shorter than 10 days.

The terms "combinatorial treatment", "combination therapy" and "combined treatment" are used interchangeably herein.

According to the present invention also pharmaceutically acceptable salts of valproic acid can be used for combinatorial therapy of cancer.

Valproic acid or a pharmaceutically acceptable salt thereof is used for the manufacture of a medicament to sensitize human cancer cells for treatment efficacy in combination therapy with clinically established anti-cancer therapeutic agents. Cancer cells are sensitized upon contact with a sensitizing agent when a lower dose of a given anti-cancer agent is required to achieve a certain anti-cancer effect compared with cancer cells which have not been contacted with said sensitizing agent. Anti-cancer effects may be reduction in tumor mass, inhibition of proliferation and/or cytotoxicity. Methods to determine anti-tumor effects are known to those skilled in the art.

According to the present invention, VPA or pharmaceutically acceptable salts thereof are used for a combinatorial treatment of human cancer or for the manufacture of a medicament for a combinatorial treatment of human cancer. The combinatorial treatment may comprise a known anti-tumor therapy.

For those cancers which manifest themselves as solid tumors, the most efficient way of treatment is to surgically remove the tumor mass. For early stage tumors, which are completely resectable, drug therapy is infrequently used. At later stages however, the tumor has usually grown to such a size and/or has spread through the body to such an extent that resection is no longer a suitable treatment option. In these cases, drug therapy is used either to reduce the size of the tumor before resection, or to eliminate residual cancer cells (minimal residual disease) in the body after tumor resection.

Today, there are different classes of anti-cancer drug therapies including chemotherapeutic and cytotoxic reagents, differentiation-inducing reagents (e.g. retinoic acid, vitamin D, cytokines), hormonal therapy, immunological approaches and, more recently, the development of anti-angiogenic approaches.

Valproic acid and pharmaceutically acceptable salts thereof exhibit a HDAC inhibitory activity and frequently and unexpectedly cause a synergistic therapeutic effect upon combinatorial therapy with one or several other anti-cancer treatments which target mechanisms strikingly different from each other.

Valproic acid or a pharmaceutically acceptable salt thereof is usually capable of sensitizing human cancer cells. Therefore, valproic acid or a pharmaceutically acceptable salt thereof is also termed the sensitizing agent herein. As a consequence, the dosage of the second therapeutic agent in a combination therapy can be signifcantly reduced to achieve an anti-tumor effect when used in combination with the sensitizing agent. The dosage of the second therapeutic agent preferably can be reduced by at least 25% compared to the dosage usually administered in clinical anti-cancer therapy. More preferably, the dosage can be reduced by at least 50%. The "dosage usually administered in clinical anti-cancer therapy" is defined herein as the amount of anti-cancer agent per sm (m²) or kg body weight (BW) of the patient per day (for references and dosing details see also S. Seeber and J. Schütte, Therapiekonzepte Onkologie, Springer-Verlag, 2. Auflage 1998, ISBN 3-540-58586-9).

Commonly used anti-cancer agents and daily dosages usually administered include
- Radiation: 20-60 Gy

The daily dosages of radiation can be significantly reduced in a combinatorial treatment with the sensitizing agent, compared to their usual dosages when administered alone or with other therapeutic principles. The following daily dosages may be used in the combinatorial treatment according to the invention:
- Radiation: 10-45 Gy

Valproic acid or a pharmaceutically acceptable salt thereof may be useful for inhibiting mammalian (for use of cell lines in in vitro assays and animal models systems) and in particular human (in vivo and in vitro) histone deacetylases HDAC 1-3 and 8 (class I), HDAC 4-7 (class II), as well as a recently identified new class of histone deacetylases with homology to the yeast SIR2 protein including several putative mammalian members (lmai et al., 2000, Nature 403, 795-800) and for the use in cancer treatment in combination with other cancer therapies. In one embodiment valproic acid or a pharmaceutically acceptable salt thereof inhibits only a subset of HDACs.

Yet another aspect of the invention is the use of valproic acid or a pharmaceutically acceptable salt thereof for the manufacture of a medicament for the combinatorial treatment of a disease in which the induction of hyperacetylation of histones has a beneficial effect, e.g. resulting in differentiation and/or apoptosis of a patient's tumor cells and thus causing a clinical improvement of the patient's condition. Examples of such diseases are estrogen receptor-dependent and independent breast cancer, colon cancer, head and neck cancer, small cell lung carcinoma. The combinatorial treatment of the present invention is particularly useful for treating minimal residual tumor disease or tumor metastases.

The compounds and salts thereof can be formulated as pharmaceutical compositions (e.g. powders, granules, tablets, pills, capsules, injections, solutions, foams, enemas and the like) comprising at least one such compound alone or in admixture with pharmaceutically acceptable carriers, excipients and/or diluents. The pharmaceutical compositions can be formulated in accordance with a conventional method. Specific dose levels for any particular patient will be employed depending upon a variety of factors including the activity of specific compounds employed, the age, body weight, general health, sex, diet, time of administration, route of administration, rate of excretion, drug combination, and the severity of the particular disease undergoing therapy. The sensitizing agent will preferably be administered in an appropriate amount, for example, selected from the range of 10 mg/kg to 100 mg/kg body weight a day orally or intravenously. The dose levels are not specifically restricted as long as serum levels of 0.05 mM to 3 mM, preferably of 0.4 mM to 1.2 mM are achieved.

In general the present invention provides novel possibilities to treat various cancer diseases. Applicant found that the HDAC inhibitory and cellular differentiation-inducing activity of VPA and pharmaceutically acceptable salts thereof can be used successfully in combination with well established and clinically used therapeutic drugs for the treatment of tumor cells of different origins. Such VPA and pharmaceutically acceptable salts thereof based combinatorial treatment is considered to generate superior therapeutic success in human tumor patients than the corresponding therapeutic drugs used on their own. It is an object of the present invention to provide combinatorial therapeutic approaches using VPA and pharmaceutically acceptable salts thereof for the treatment of cancer. Such combinatorial treatments could result in a decrease of the therapeutic doses of e.g. chemotherapeutic reagents required and could thus limit the currently observed, partly very serious side effects of frequently used therapies.

Aspects of the present invention are the combination of VPA or pharmaceutically acceptable salts thereof with radiation therapy (e.g. x-rays or gamma rays).

After tumor therapy often residual tumor cells remain in the patients' bodies. This condition is known as minimal residual disease. These tumor cells can give rise to secondary tumors even years after the primary tumor has been removed. Therefore, one major task of a successful tumor therapy must be the eradication of such residual tumor cells.
Thus, another aspect of the invention is the use of VPA and pharmaceutically acceptable salts thereof for the inhibition of tumor metastasis and therefore the extinction of minimal residual disease.

We tested the effect of irradiation in combination with VPA on human tumor cells of various origins. Surprisingly we regularly found that the combination of VPA with irradiation had a synergistic anti-tumor effect compared to the effects seen with VPA alone or irradiation alone. These frequently observed enhancements were unexpected.
The most likely basis for this therapeutic success of VPA is its activity as a novel inhibitor of enzymes having HDAC activity. However, since e.g. TSA does not display this synergistic activities to the same extent VPA does, it appears that the fine tuned mechanistic targeting achieved by VPA appears to be superior to other HDAC inhibitors. In addition, VPA may be employed for the inhibition of tumor metastases formation and thus for the treatment of minimal residual disease. This was successfully tested by using in vivo models of renal and breast carcinomas.

**Figure 1** shows the effect of VPA on the differentiation block of hematopoietic progenitors in vitro: VPA cooperates with cytokines in restoring the differentiative potential of PML-RAR expressing cells (reference example) and must therefore be regarded as a sensitizing and synergistically acting reagent for the differentiation inducing activity of cytokines.

**Figure 2** shows that VPA sensitizes PML-RAR expressing cells to X-ray treatment in vitro (Example 1) and causes synergistic therapeutic activities in this combination.

The following examples further illustrate the invention:

### Reference example

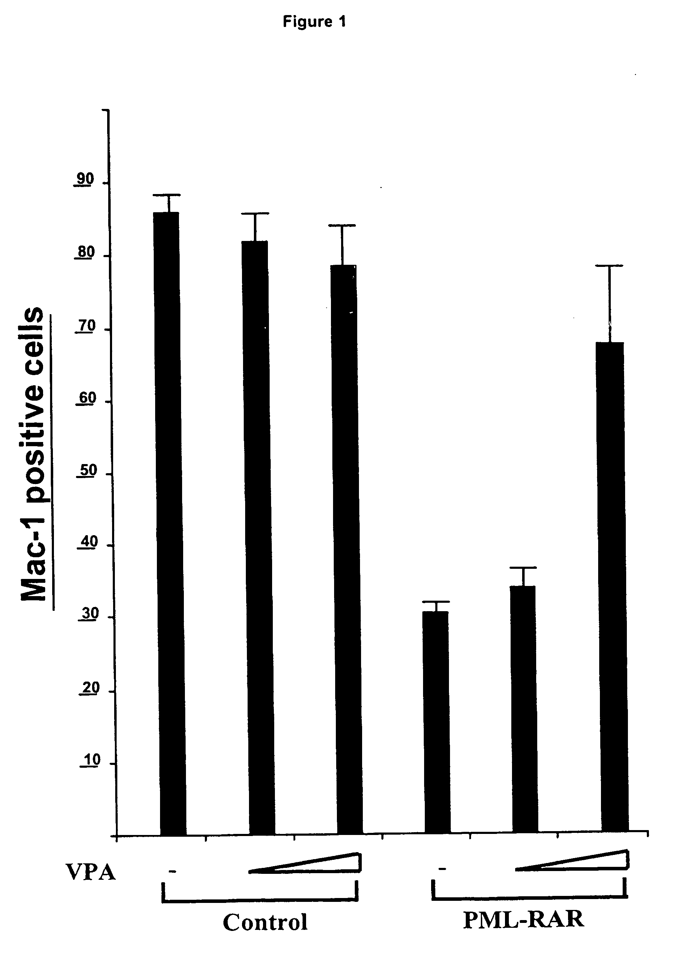

Synergistic effect of VPA on the differentiation block of hematopoietic progenitors in vitro: VPA cooperates with cytokines in restoring the differentiative potential of PML-RAR expressing cells. VPA must therefore be regarded as a sensitizing reagent for the differentiation inducing activity of cytokines (Figure 1).

Since acute promyelocytic leukemia cells are known to respond to treatment with HDAC inhibitors, the effect of VPA on the differentiation block of hematopoietic precursor cells by PML-RAR was tested. Murine hematopoietic progenitors (lin-) were transduced with a retroviral vector encoding PML-RAR, and GFP as a marker. Transduced cells were stimulated to differentiate with a cocktail containing several cytokines (IL3, IL6, SCF, G-CSF and GM-CSF) in the absence or presence of VPA. Myeloid differentiation was scored by analyzing the presence of the differentiation marker Mac-1.

### Results:

VPA treatment did not affect differentiation of control cells whereas expression of PML-RAR caused a strong differentiation block (Figure 1) which could not be overcome when the cytokines described where used as the only treatment. However, VPA (1 mM, in right panels, labeled PML-RAR) almost completely reverted the differentiation block imposed by PML-RAR (Figure 1). Thus, in the absence of RA, VPA re-establishes and sensitizes the cells for a state permissive for differentiation, then induced by cytokines, presumably by inhibiting the action of the HDAC complex recruited to target genes by PML-RAR. This activity of VPA must be regarded as a synergistic activity since the treatment with cytokines alone does not lead to a significant release of the differentiation block in these PML-RAR cells as mentioned above.

### Methods:

Murine hematopoietic progenitors were purified from the bone marrow of 129 mice on the basis of the absence of lineage differentiation markers (lin-). Lin- cells were grown for 48 hours in the presence of IL-3 (20 ng/ml), IL-6 (20 ng/ml), SCF (100 ng/ml), and then attached to non-tissue culture treated plates coated with Retronectin (Takara-Shuzo). Cells were then transduced by incubation with the supernatant from Phoenix ecotropic packaging cells (supplemented with fresh serum, and IL-3, IL-6, and SCF as above), transiently transfected with the control retroviral vector PINCO, or PINCO-PML-RAR. After 60 hours, GFP+ cells were sorted by FACS, and seeded in methylcellulose plates supplemented as above, plus G-CSF (60 ng/ml) and GM-CSF (20 ng/ml). Where indicated, sodium valproate (VPA, from left to right 0.2 or 1mM) was added to the differentiation medium. After 8-10 days, cells were analyzed for the presence of the myeloid differentiation marker Mac-1 by FACS. VPA did not cause significant changes in the number of Mac-1⁺ cells, nor in the number of colonies in control cells up to concentrations of 1mM. At higher concentrations (>3 mM) a reduction in the number of colonies was observed, most likely due to induction of cell death (data not shown). As a control, the analysis of VPA-treated cells with an erythroid differentiation marker (Ter-119) did not reveal the presence of positive cells (data not shown). Uninfected cells, and cells infected with the control PINCO vector behaved identically (data not shown).

For figure 1 Lin- cells were transduced with the indicated vectors (control: PINCO, empty vector encoding GFP alone), and GFP+ cells were sorted by FACS. GFP+ cells were then plated in differentiation medium, in the absence or in the presence of VPA (from left to right 0.2 and 1mM). Differentiation was assessed after 8-10 days by analysis of the myeloid differentiation marker Mac-1.

### Example 1

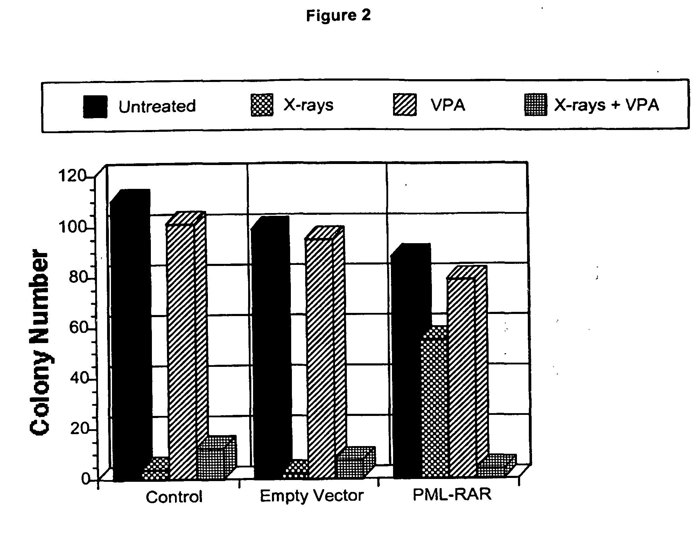

VPA sensitizes PML-RAR expressing cells to X-ray treatment in vitro and causes a synergistic therapeutic effect (Figure 2).

### Results:

Upon X-ray treatment (2 Gray), hematopoietic progenitor cells show a strong decrease in their survival potential and undergo apoptosis. Semisolid culture conditions (methylcellulose-based mediums) led to an almost complete absence of colonies (derived from colony-forming cells, CFCs) in X-rays treated cultures of wild-type cells, demonstrating that undifferentiated cells are very sensitive to this treatment (Figure 2). Strikingly, PML-RAR expression caused a strong reduction in X-ray sensitivity of target cells, with a >50% rescue rate (Figure 2). Under the same conditions, VPA (1mM) slightly decreased the sensitivity of wild-type cells. However, VPA led to a re-sensitization of PML-RAR expressing cells, with a complete and clearly synergistic disappearance of colonies in VPA treated cells (Figure 2). It appears therefore, that VPA may be combined with X-rays to rescue the sensitivity of cells that have become resistant (e.g. through expression of an oncogenic fusion protein) to X-ray treatment alone and may cause synergistic therapeutic success rates.

### Methods:

Lin- cells were transduced as described for figure 1 ( see also methods in the reference example for details), and sorted by FACS. 12 hours after sorting, cells were washed with PBS, and then incubated for 8-12 hours in medium with cytokines [data presented in figure 2 using IL-3 (20 ng/ml), IL-6 (20 ng/ml), SCF (100 ng/ml), G-CSF (60 ng/ml), GM-CSF (20 ng/ml)] or without cytokines (data not shown). At the end of the incubation, cells were exposed to an X-ray source (2 Gy total exposure), and incubated for additional 12-16 hours in the presence or in the absence of VPA. Finally, cells were plated in methylcellulose containing medium (StemCell Technologies) in the presence of cytokines (IL3, IL6, SCF, G- and GM-CSF) for 8-10 days.

For Figure 2 uninfected cells ("Control"), or cells expressing GFP alone ("Empty Vector"), or cells expressing GFP and PML-RAR ("PML-RAR") were used. 8 days after plating in methylcellulose, the total number of colonies was measured. VPA-treated cells (1mM) were exposed to VPA also prior to X-ray treatment.

## Claims

1. The use of valproic acid or a pharmaceutically acceptable salt thereof for the manufacture of a medicament for the treatment of human cancer in combination with irradiation treatment, wherein the human cancer is selected from the group consisting of breast cancer, colon cancer, head and neck cancer, small cell lung carcinoma and cancer of blood cells.

2. A use according to claim 1 wherein the combination treatment further includes anti-tumor therapy selected from the group consisting of treatment with differentiation inducing drugs, treatment with chemotherapeutic drugs, treatment with cytotoxic drugs, hormone therapy, immunotherapy, anti-angiogenic therapy and/or gene therapy.

3. A use according to claim 1 or 2 wherein valproic acid is an inhibitor of enzymes having HDAC activity and causes an additive or synergistic therapeutic effect.

4. A use according to claim 3 wherein the enzyme having histone deacetylase activity is a mammalian, preferably a human histone deacetylase.

5. A use according to claim 3 or 4 wherein the human histone deacetylase is selected from the group consisting of HDACs 1-8 and members of the SIR2 protein family.

6. A use according to anyone of claims 3 to 5 wherein valproic acid specifically inhibits only a subset of HDACs.

7. A use according to anyone of claims 1 to 6 wherein valproic acid is used for the induction of differentiation of cells.

8. A use according to anyone of claims 1 to 6 wherein valproic acid is used for the induction of differentiation of transformed cells.

9. A use according to anyone of claims 1 to 6 wherein valproic acid is used for the induction of apoptosis of transformed cells.

10. A use according to anyone of claims 1 to 9 wherein the induction of hyperacetylation of histones or other proteins functionally regulated by acetylation has a beneficial effect for the treatment of human cancer.

11. A use according to anyone of claims 1 to 10 wherein valproic acid or a pharmaceutically acceptable salt thereof is tube administered as a first therapeutic agent, and V radiation therapy is tube administered as a second therapeutic agent, **characterized in that** the daily dosage of said radiation therapy is significantly reduced compared to the daily dosage of the radiation therapy when administered alone.

## Patentansprüche

1. Verwendung von Valproinsäure oder einem pharmazeutisch annehmbaren Salz davon zur Herstellung eines Arzneimittels zur Behandlung von Krebs beim Menschen in Kombination mit einer Strahlenbehandlung, wobei der Krebs ausgewählt ist aus der Gruppe bestehend aus Brustkrebs; Dickdarmkrebs, Kopf- und Halskrebs, kleinzelligem Lungenkarzinom und Blutzellenkrebs.

2. Verwendung nach Anspruch 1, wobei die Kombinationsbehandlung weiterhin eine Antitumortherapie einschließt ausgewählt aus der Gruppe bestehend aus der Behandlung mit die Differenzierung induzierenden Wirkstoffen, der Behandlung mit chemotherapeutischen Wirkstoffen, der Behandlung mit cytotoxischen Wirkstoffen, Hormontherapie, Immuntherapie, antiangiogener Therapie und/oder Gentherapie.

3. Verwendung nach Anspruch 1 oder Anspruch 2, wobei Valproinsäure ein Inhibitor von Enzymen mit HDAC-Aktivität ist und einen additiven oder synergistischen therapeutischen Effekt bewirkt.

4. Verwendung nach Anspruch 3, wobei das Enzym mit Histondeacetylaseaktivität eine Histondeacetylase vom Säugetier, bevorzugt vom Menschen ist.

5. Verwendung nach Anspruch 3 oder Anspruch 4, wobei die Humanhistondeacetylase ausgewählt ist aus der Gruppe bestehend aus HDACs 1 bis 8 und Mitgliedern der SIR2-Proteinfamilie.

6. Verwendung nach einem der Ansprüche 3 bis 5, wobei die Valproinsäure spezifisch nur eine Untergruppe von HDACs hemmt.

7. Verwendung nach einem der Ansprüche 1 bis 6, wobei Valproinsäure zur Induktion der Differenzierung von Zellen verwendet wird.

8. Verwendung nach einem der Ansprüche 1 bis 6, wobei Valproinsäure zur Induktion der Differenzierung von transformierten Zellen verwendet wird.

9. Verwendung nach einem der Ansprüche 1 bis 6, wobei Valproinsäure zur Induktion der Apoptose von transformierten Zellen verwendet wird.

10. Verwendung nach einem der Ansprüche 1 bis 9, wobei die Induktion der Hyperacetylierung von Histonen oder von anderen Proteinen, die funktionell durch Acetylierung reguliert werden, eine vorteilhafte Wirkung auf die Behandlung von Krebs bei Menschen hat.

11. Verwendung nach einem der Ansprüche 1 bis 10, wobei Valproinsäure oder ein pharmazeutisch annehmbares Salz davon als erstes therapeutisches Mittel zu verabreichen ist und die Strahlentherapie als zweites therapeutisches Mittel zu verabreichen ist, **dadurch gekennzeichnet, dass** die tägliche Dosis der Strahlentherapie signifikant reduziert ist im Vergleich zu der täglichen Dosis der Strahlentherapie, wenn diese allein angewandt wird.

## Revendications

1. Utilisation d'acide valproïque ou d'un sel pharmaceutiquement acceptable de celui-ci pour la fabrication d'un médicament destiné au traitement d'un cancer humain en combinaison avec un traitement par rayonnement, dans laquelle le cancer humain est sélectionné parmi le groupe constitué du cancer du sein, cancer du colon, cancer de la tête et de cou, carcinome à petites cellules du poumon et cancer de cellules sanguines.

2. Utilisation selon la revendication 1, dans laquelle le traitement combiné comporte de plus une thérapie anti-tumorale sélectionnée parmi le groupe constitué d'un traitement par des médicaments induisant une différenciation, traitement par des médicaments chimiothérapeutiques, traitement par des médicaments cytotoxiques, thérapie hormonale, immunothérapie, thérapie anti-angiogénique et/ou thérapie génique.

3. Utilisation selon la revendication 1 ou 2, dans laquelle l'acide valproïque est un inhibiteur d'enzymes ayant une activité HDAC et provoque une effet thérapeutique additif ou synergique.

4. Utilisation selon la revendication 3, dans laquelle l'enzyme ayant une activité histone désacétylase est une histone désacétylase mammifère, de préférence humaine.

5. Utilisation selon la revendication 3 ou 4, dans laquelle l'histone désacétylase humaine est sélectionnée parmi le groupe constitué des HDAC 1-8 et de membres de la famille des protéines SIR2.

6. Utilisation selon l'une quelconque des revendications 3 à 5, dans laquelle l'acide valproïque inhibe de manière spécifique uniquement un sous-ensemble des HDAC.

7. Utilisation selon l'une quelconque des revendications 1 à 6, dans laquelle l'acide valproïque est utilisé pour l'induction de différenciation de cellules.

8. Utilisation selon l'une quelconque des revendications 1 à 6, dans laquelle l'acide valproïque est utilisé pour l'induction d'une différenciation de cellules transformées.

9. Utilisation selon l'une quelconque des revendications 1 à 6, dans laquelle l'acide valproïque est utilisé pour l'induction d'une apoptose de cellules transformées.

10. Utilisation selon l'une quelconque des revendications 1 à 9, dans laquelle l'induction d'une hyperacétylation d'histones ou autres protéines régulées de manière fonctionnelle par acétylation a un effet bénéfique pour le traitement d'un cancer humain.

11. Utilisation selon l'une quelconque des revendications 1 à 10, dans laquelle l'acide valproïque ou un sel pharmaceutiquement acceptable de celui-ci est à administrer en tant que premier agent thérapeutique, et une thérapie par rayonnement est à administrer en tant que second agent thérapeutique, **caractérisée en ce que** la dose quotidienne de ladite thérapie par rayonnement est nettement réduite par comparaison à la dose quotidienne de la thérapie par rayonnement lorsque administrée seule.
